# EUROPEAN PATENT SPECIFICATION

(11) **EP 1 752 767 B1**
(45) Date of publication and mention of the grant of the patent: **13.07.2011**
(21) Application number: 05739187.2
(22) Date of filing: 10.05.2005
(51) Int. Cl.: G01N 33/53, G01N 21/82, G01N 33/543, G01N 33/545

(54) **METHOD OF ASSAYING HYALURONIC ACID BY USING HYALURONIC ACID-BINDING PROTEIN**
VERFAHREN ZUM TESTEN VON HYALURONSÄURE MIT HYALURONSÄURE-BINDUNGSPROTEIN
PROCÉDÉ DE DOSAGE DE L'ACIDE HYALURONIQUE EN UTILISANT UNE PROTÉINE LIANT L'ACIDE HYALURONIQUE

(30) Priority: 20.05.2004 JP 2004149940
(43) Date of publication of application: 14.02.2007
(73) Proprietor: Wako Pure Chemical Industries, Ltd., Osaka-shi, Osaka 540-8605 (JP)
(72) Inventor: SUMIDA, Kyoichi, 6610963 (JP); FUJIO, Kazunari, 6610963 (JP); KOBATAKE, Shinzo, 6610963 (JP)
(74) Representative: von Kreisler Selting Werner
(86) International application number: PCT/JP2005/008523
(87) International publication number: WO 2005/114186

(56) References cited:
- EP-A1- 1 329 717
- WO-A-02/101389
- JP-A- 8 193 999
- JP-A- 11 014 628

## Description

### Technical Field

The present invention relates to a method for measuring hyaluronic acid having good storage stability and affording high degree of accuracy in measurement and a reagent kit for the same.

### Background Art

Hyaluronic acid is present mainly in the synovial fluid and ocular vitreous humor of animal and connective tissue such as the umbilical cord and the upper dermis of animals. The concentration of hyaluronic acid in the blood is known to increase in the patients with rheumatoid arthritis, cancer and liver disease and known as a useful marker for diagnosis of these diseases, and therefore, various methods for measuring hyaluronic acid have been developed to date. For example, WO02/10138A presents a homogenous assay method for detection of hyaluronic acid avoiding the use of a solid phase. The method relies on a labeled hyaluronic acid binding protein.

On the other hand, for immunological measurement, methods of utilizing latex particles have been used widely because of its simple procedure and applicability to multipurpose measuring devices. JP-A-8193999 relates to an immunoassay method directed at general detection of antibody/antigen interactions. In particular, an immuno complex I formed by antibody/antigen, e.g. in a blood sample is detected by a further specific antibody, which may be supported on an insoluble carrier. The application to hyaluronic acids is not mentioned. JP-A-11014628 presents an assay method designed to avoid reliance on antibody/antigen interactions. In one embodiment, a hyaluronic acid binding protein is directly immobilized on a carrier, and biological sample (e.g. blood) can be assayed for hyaluronic acid. The method employing latex particles as a reagent for measuring hyaluronic acid has been described, for example, in JP-B-3424504. In the aforementioned patent publication, a method comprising processes of supporting a hyaluronic acid binding protein on carrier particles, forming a reaction complex between the particles and hyaluronic acid in a sample and determining the hyaluronic acid by detecting the reaction complex has been described. In Examples of the aforementioned patent publication, experiments have been carried out using latex particles with average particle size of 368 nm in diameter. However, when such a particles is used as a reagent, usually, the latex particles has to be dispersed by shaking or the like before use, because the latex particles with average particle size of 300 nm (0.3 µm) or larger in diameter have a tendency to be easily deposited. Therefore, for the measurement of hyaluronic acid using a carrier such as latex particles, the development of a reagent, which is not deposited easily and does not need a cumbersome treatment such as shaking before use, has been desired.

Patent Literature 1: JP-B-3424504
Patent Literature 2: WO 02/101389A
Patent Literature 3: JP-A-8 193999
Patent Literature 4: JP-A-11014628A

### Disclosure of the Invention

### Problem to be Solved by the Invention

On the view of above situation, an object of the present invention is to provide a method utilizing a carrier for measuring hyaluronic acid wherein deposition of the carrier is less, storage stability is good and accuracy in measurement has the same high degree as that performed by the conventional reagents, and a reagent kit for the same.

### Means for Solving the Problem

To store a reagent containing latex particles for the measurement of hyaluronic acid in a more stable manner, the present inventors have investigated to find a method of supporting a hyaluronic acid binding protein (hereinafter, optionally referred to as HABP) on latex particles with less deposition (average particle size: 0.3 µm or less in diameter) by chemical bond or physical adsorption. However, it was difficult to efficiently support HABP on latex particles, and therefore, the measurement could not be performed with a high degree of accuracy. Then, as the results of intensive study, the inventors have found that the agglutination reaction of latex particles in accordance with the quantity of hyaluronic acid can be induced effectively by the reaction of latex particles, which have preliminarily been sensitized with a monoclonal antibody for HABP, with a complex formed between HABP and hyaluronic acid. Furthermore, the present inventors have found that a reagent for the measurement of hyaluronic acid with good storage stability can be prepared by storing the latex particles which was preliminarily sensitized with a monoclonal antibody for HABP and the hyaluronic acid binding protein individually as separate reagents. Furthermore, they have found that, as described above, the step can be proceeded by forming a complex preliminarily by reacting hyaluronic acid with HABP and then by reacting the complex with the latex particles sensitized with a monoclonal antibody for HABP, and thus completed the present invention.

That is, the present invention relates to "a method for measuring hyaluronic acid comprising forming a hyaluronic acid/HABP complex by contacting hyaluronic acid in a sample with HABP, reacting said complex with an anti-HABP antibody supported carrier, measuring the optical change of agglutination product generated by said reaction, and calculating the quantity of hyaluronic acid from the measured value" and "a reagent kit for the measurement of hyaluronic acid comprising a reagent comprising HABP and a reagent comprising an anti-HABP antibody supported carrier".

### Effect of the Invention

According to the measurement method of the present invention, in comparison with conventional method, the measurement can be performed by a simple procedure such as without need for shaking of the reagent before use, and also, the measurement can be performed with the same high degree of accuracy as that by conventional reagents. In addition, the reagent kit of the present invention provides less deposition of carrier and good storage stability; in addition, using said reagent kit, measurement of hyaluronic acid can be achieved with the same degree of accuracy as that by conventional reagents

### Best Mode for Carrying Out the Invention

As the hyaluronic acid binding protein (HABP) in regard to the present invention, any one containing a hyaluronic acid binding region of proteins which have the property to bind hyaluronic acid such as proteoglycan, link protein, and hyaluronectin may be adopted without specific limitation, and it may include above described protein itself, a partial protein containing hyaluronic acid binding region of the above described protein, a substance containing such partial protein and a recombination protein produced by incorporating a gene fragment encoding hyaluronic acid binding region of the above described protein into another protein.

As an anti-HABP antibody in regard to the present invention, any antibody against HABP, whether it is monoclonal or polyclonal, may be adopted. A polyclonal or a monoclonal antibody, purified by affinity chromatography with a single epitope is preferable, and a monoclonal antibody capable of binding efficiently with hyaluronic acid is particularly preferable. Among these, the use of Fab, Fab' and F(ab')₂ produced by appropriate digestion of these antibodies using an enzyme such as pepsin and papain is preferable. When a polyclonal antibody is used as anti-HABP antibody, the antibody can be prepared by a conventional method of immunization of an animal such as horse, cow, sheep, rabbit, goat, rat or mouse with hyaluronic acid binding protein according to the methods described, for example, in "Matsuhashi, T. et al., Introduction to Experimental Immunology, 2nd ed., 1981, Japan Scientific Societies Press". When a monoclonal antibody is used as anti-HABP antibody, the antibody can be prepared according to a conventional method, namely, the cell fusion technology established by Kohler and Milstein (G. Kohler and C. Milstein: Nature, 256, 495 (1975)), for example, using a hybridoma cell obtained by fusing a cell line derived from mouse myeloma with cells from the mouse spleen which is preliminarily immunized with hyaluronic acid binding protein.

As a carrier in regard to the present invention, any carrier usually used in the immunological measurement can be adopted, specifically as preferable ones, carriers prepared from, for example, natural organic polymer substances such as red blood cells, bacteria and cell fragments, assemblies of molecule such as liposomes and polymeric micelles, synthetic polymer compounds such as polystyrene, polyacrylic acid, polymethacrylic acid, polyacrylamide, polyglycidylmethacrylate, polypropylene, polyvinylchloride, polyethylene, polychlorocarbonate, silicone resin and silicone rubber, inorganic substances such as porous glass, ground glass, alumina, silica gel, activated carbon and metal oxide are included. In addition, these carriers can be used in various forms such as tube, bead, disc type chip, micro particle or latex particle. Among them, the latex particle is particularly preferable from the point of view, for example, that chemical treatment of the surface of the carrier can be easily carried out as appropriate for any purpose because the carrier material is an artificial polymer and that nonspecific reaction hardly takes place. As to the quality of material, it has no specific limitation, but preferably includes, for example, a styrene type latex particle such as polystyrene latex particle and an acrylic acid type latex particle.

In this connection, among these latex particles, polystyrene latex particles and the like which are prepared by emulsion polymerization reaction without use of emulsifying agent are particularly preferable. As they have a surface with strong hydrophobic nature, proteins or peptides can be adsorbed smoothly, and as they have a negatively charged surface and cause mutual repulsion between them, they can stably disperse in a solution even in the absence of emulsifying agent. In addition, various modified latex particle (for example, a carboxylic acid modified latex particle produced by introducing carboxyl group into the above described polystyrene), a magnetic latex particle (a magnetic particle-encapsulated latex particle) can be used as well.

In addition, as for latex particles to be used in the present invention, commercially available latex with small average particle diameter, namely, with large surface area per unit weight, is able to support an antibody efficiently and also provides good storage stability (good dispersibility in a solution), and so, it is used preferably. More specifically, the average particle diameter is usually 0.05 to 0.3 µm, preferably 0.1 to 0.25 µm. Using such latex particles with small average diameter, deposition of the particle can be avoided and it can be achieved to efficiently support an anti-HABP antibody on the latex particles. That is, by the use of such anti-HABP antibody-supported latex particles, both the increased stability of the measurement reagent and high accuracy of measurement can be achieved.

The method of supporting an anti-HABP antibody involved in the present invention on a carrier involved in the present invention may be performed without specific limitation by contacting the anti-HABP antibody with the carrier. All the supporting methods well known per se usually used in this field can be included, and for example, as an exemplary method, a method of supporting the anti-HABP antibody on the carrier by physical adsorption, so called physical adsorption method (refer to, JP-A-1993-41946; SUMILON Technical Report, SUMILON ELISA series 1 Introduction to ELISA Method, published by Sumitomo Bakelite Co., Ltd.; SUMILON Technical Report, SUMILON ELISA series 2 Solid Phase Surface of ELISA Products, published by Sumitomo Bakelite Co., Ltd., and so on) is included as representative examples. The aforementioned method is usually used as a preferable method when, for example, synthetic polymer compounds such as polystyrene, polypropylene, polyvinylchloride, polyethylene, polychlorocarbonate; activated carbon; inorganic substances such as porous glass, ground glass, alumina, silica gel and metal oxide hydroxy apatite are used as carrier. Among them, it is particularly preferable when glass, polystyrene and polyvinylchloride are used in the form of, for example, tube, bead, disc chip, micro particle or latex particle.

For example, when an anti-HABP antibody involved in the present invention is supported on the latex particles, the latex particles are added so as to be present in a concentration of usually 0.1 to 10%(w/v), preferably 0.2 to 5% (w/v) and suspended in a solvent such as a buffer solution containing usually 0.05 to 2 mg/ml, preferably 0.1 to 1 mg/ml of an anti-HABP antibody involved in the present invention. After reacting usually at 5 to 30°C and usually for 2 to 3 hours, post treatments usually conducted in this field such as, centrifugation, blocking treatment using a solution containing an appropriate protein such as bovine serum albumin (BSA) are carried out, thus working up the supporting process. In this connection, it can also be achieved to support the anti-HABP antibody on a carrier by chemical binding methods usually used in this field.

The method for measuring hyaluronic acid of the present invention may be performed by the process wherein a hyaluronic acid/HABP complex is formed by contacting hyaluronic acid in a sample with HABP, followed by reacting the said complex with anti-HABP antibody supported carrier, followed by measuring optical change of agglutination product generated by said reaction and followed by calculating the quantity of hyaluronic acid from the measured value.

In this connection, the measurement of the optical change described herein means the measurement of the optical change caused by the formation of immunoagglutination, and more specifically, in this category, immunoagglutination methods such as reversed passive agglutination method, nephelometric immunoassay and turbidimetric immunoassay are included. These measurement methods may be performed according to the method well known per se. When the reversed passive agglutination method is employed, the method may be carried out according to the procedure described, for example, in "Successive Course on Biochemical Experiment 5: Investigative Approach to Immunobiochemistry", Tokyo Kagaku Dojin Co., Ltd., pp. 36-37, "A Manual of Clinical Laboratory Method", 30th ed., Kanehara & Co. , Ltd., pp. 844-845, and when nephelometric immunoassay is employed, the method may be carried out according to the procedure described, for example, in "A Manual of Clinical Laboratory Test", 30th ed., Kanehara & Co., Ltd., pp. 851-853, and when turbidimetric immunoassay is employed, the method may be carried out according to the procedure described, for example, in "A Manual of Clinical Laboratory Method", 30th ed., Kanehara & Co. , Ltd. , pp. 853-854.

Taking the turbidimetric immunoassay using latex particle carrier as an example, the measuring method of the present invention will be described more specifically below. That is, a sample containing hyaluronic acid (more specifically, for example, body fluid such as blood, plasma, serum, synovial fluid, pleural fluid, lymph fluid, spinal fluid and urine) is contacted and mixed with a reagent containing above described HABP to form hyaluronic acid/HABP complex. Then, for example, a reagent, wherein above described anti-HABP antibody is supported (sensitized) on latex particles with average particle diameter of, for example, 0.05 to 0.3 µm, preferably 0.1 to 0.25 µm, is reacted with above described complex. The degree of resulting agglutination is measured, for example, by means of absorbance, and the concentration is determined from a calibration curve preliminarily prepared using standard sample, and thus, the quantity of hyaluronic acid in a sample is assayed. In this connection, absorbance measurement may be carried out usually at wavelength of 340 to 1000 nm, preferably at 500 to 900 nm. In addition, determination of the degree of agglutination is not limited to the measurement of absorbance; the degree may be measured by any method well known per se, for example, by nephelometry or by counting immunoassay. In addition, when the hyaluronic acid/HABP complex is reacted with a reagent containing an anti-HABP antibody which has been supported on a carrier such as latex particles (hereinafter, may referred to as anti-HABP antibody-supported carrier), an appropriate agglutination accelerating agent may be added. In this connection, specific examples of such agglutination-accelerating agent will be described in the section of "reagent kit" in the present invention.

In the measurement method of the present invention, the use concentration of HABP in the HABP reactions is, although it may vary depending on the detection limit of hyaluronic acid be set out, usually, equal to or higher than the concentration which is capable of binding with the whole amount of hyaluronic acid that corresponds to the set concentration of the detection limit, preferably 5 times or more, more preferably 10 times or more of the set concentration of determination limit. In this connection, the upper limit concentration of hyaluronic acid on this occasion has no limitation, in consideration of economical amount of the hyaluronic acid, the concentration is usually 50,000 times or less, preferably 10,000 times or less. Specifically, the concentration is usually from 0.1 to 1000 µg/ml, preferably from 0.5 to 1000 µg/ml, and more preferably from 0. 5 to 100 µg/ml. For example, when hyaluronic acid concentration in serum is measured, as usual determination limit is from 10 to 1000 ng/ml. The use concentration of HABP in HABP reaction may, therefore, be set out appropriately within the above-described range based on the determination limit.

In addition, as to the pH in the aforementioned reaction, the range thereof is not specifically limited as long as it does not inhibit formation of the complex, and is usually 5 to 10, preferably 6 to 8. Also, as to the temperature in the aforementioned reaction, the range thereof is not specifically limited as long as it does not inhibit the formation of the complex, and is usually 5 to 40°C. In addition, as the reaction time may differ according to the reaction condition such as HABP used and pH and temperature, the reaction may be conducted for several seconds to several hours as appropriate according to each condition.

In the measurement method of the present invention, the use concentration of anti-HABP antibody-supported carrier in the reaction between anti-HABP antibody-supported carrier and hyaluronic acid/HABP complex is, while it may vary depending on the use concentration of HABP in the above reaction, usually 0. 2 to 25 mg/ml, preferably 0.5 to 12 mg/ml when latex particles having 0.01 to 0.1 mg/mg of supporting amount of anti-HABP antibody are used, and, if it is within the range of aforementioned concentration, the hyaluronic acid in a sample can be measured with a high degree of accuracy. In this connection, the condition and the time for the reaction of anti-HABP antibody-supported carrier with hyaluronic acid/HABP complex may be adopted in accordance with that for the above mentioned HABP reaction.

As a reagent kit for the measurement of hyaluronic acid of the present invention, a kit comprising both a reagent comprising HABP and a reagent comprising anti-HABP antibody-supported carrier may be included. In this connection, the aforementioned kit may contain a standard substance usually used in this field such as, for example, potassium hyaluronate (derived from cockscomb, produced by Wako Pure Chemical Industries, Ltd.) and sodium hyaluronate (derived from Streptococcus species, produced by Wako Pure Chemical Industries, Ltd.).

The reagent comprising HABP in the reagent kit for the measurement of hyaluronic acid of the present invention may be any one of reagent comprising HABP as described above, which may be dissolved in an appropriate buffer solution. As the buffering agents used for this purpose, any kind of buffering agent usually used in the immunological measurement, for example, Tris buffering agent, phosphate buffering agent, veronal buffering agent, boric acid buffering agent and Good buffering agent can be adopted, and the concentration of such buffering agent is usually 5 to 300 mM, preferably 10 to 150 mM, and the pH is usually 5 to 10, preferably 6 to 8, and the concentration and pH are each selected appropriately from above described corresponding range.

As to the concentration of HABP in the above reagent comprising HABP, while it may vary depending on the kind of HABP used, the concentration in the reaction may be set out to be the same concentration as described above, and may be selected appropriately so as to be within the range from 0.1 to 500 µg/ml, preferably 0.5 to 100 µg/ml.

In a reagent kit for the measurement of hyaluronic acid of the present invention, a reagent comprising an anti-HABP antibody-supported carrier may be any reagent containing above described anti-HABP antibody-supported carrier, which may be a suspension of anti-HABP antibody-supported carrier in an appropriate buffer solution or a lyophilized product thereof. As the buffering agents used for this purpose, any kind of buffering agent is used as long as it does not inhibit binding between anti-HABP antibody involved in the present invention and HABP, and includes the same buffering agents as used for the above described reagent comprising HABP, and also, the pH and the concentration may be set out by the same way according to the above described value.

In addition, the reagent comprising an anti-HABP antibody-supported carrier is provided in many cases in the form of a suspension suspended in a solution such as a buffer solution. As the buffer solution used for preparing such suspension, any one usually used in this field is adopted without specific limitation, and usually one having a buffering action at pH 5.0 to 10.0, preferably around neutral pH of pH 6.5 to 8.5, for example, phosphate buffer, Tris buffer or Good buffer is preferable. In this connection, depending on the characteristics of the insoluble micro particles, some one has a tendency to aggregate naturally when left in suspended condition. In such case, the use of a mildly alkaline buffer solution such as glycine buffer or boric acid buffer for the preparation of suspension is far more preferable from a standpoint of storage stability. In addition, the concentration of buffering agent in these buffers is selected appropriately from the range of usually 10 to 500 mM, preferably 10 to 300mM. In this connection, in the aforementioned reagent, for example, a stabilizing agent such as a sugar, a protein and a surface activating agent, a salt such as NaCl and an preservative substance may be added within the range usually used in this field.

When the anti-HABP antibody-supported carrier involved in the present invention is suspended in an above described buffer solution, the concentration of the anti-HABP antibody-supported carrier in the reaction may be, while it may vary depending on the kind of anti-HABP antibody used, set out to be the same concentration as described above, and may be selected appropriately so as to be usually within a range from 0.1 to 500 µg/ml, preferably from 0.5 to 100 µg/ml.

Further, in the reagent comprising an anti-HABP antibody-supported carrier involved in the present invention, an immunological reaction accelerator (agglutination reaction accelerator) (for example, polyethylene glycol and polyvinyl alcohol) may coexist at the concentration range usually used in this field, and even under coexistence of such agglutination reaction accelerator, the appearance of nonspecific turbidity of denatured protein constituent in the measuring reagent, which is caused by some sort of factor, can be repressed or reduced by the method of the present invention. In addition, a monomer or a polymer used as an agglutination accelerator described in JP-A-2002-365296 may be contained as an agglutination accelerator in the above-described reagent, and the concentration range thereof may be selected according to the value described in JP-A-2002-365296. In this connection, the aforementioned monomer or polymer may be prepared according to the method described in the above patent application.

The reagent kit for the measurement of hyaluronic acid involved in the present invention is to be used for performing such measurement method as described above of the present invention, and preferable embodiments of constituent elements and specific examples are as described above.

As to a sample involved in the present invention, any sample containing hyaluronic acid may be adopted, and specifically, it includes, for example, body fluid such as blood, plasma, serum, synovial fluid, pleural fluid, lymph fluid, spinal fluid and urine, and as preferable sample among them, serum and urine.

Some examples exemplifying the present invention are described more specifically below.

### Example 1

### (1) Preparation of the test solution

### 1. Preparation of the first test solution (HABP test solution)

One hundred (100) µg of hyaluronic acid binding protein (purified from bovine nasal septal cartilage according to a modified method of Lauren et al., product of Seikagaku Corporation) was dissolved in 10 ml of 100 mM HEPES buffer solution (containing 0.1% BSA and 1% NaCl, pH 7.0). This solution was defined as first test solution.

### 2. Preparation of second test solution (latex particles sensitized with anti-HABP monoclonal antibody)

To a 2 ml volume polycarbonate tube for centrifugation, 800 µl of purified water, 100 µl of latex particle solution (N200, product of Sekisui Chemical Co., Ltd.: 10 wt%, 220 nm of latex particle diameter), 100 µl of 500 mM boric acid buffer (pH 7.3), 100 µl of 50 mM ASES buffer solution containing anti-HABP monoclonal antibody (4.24 mg/ml, pH 6.5) were added, and incubated with stirring at room temperature for 100 minutes to obtain a suspension of anti-HABP monoclonal antibody-supported latex particles. In this connection, above described anti-HABP monoclonal antibody was prepared according to the conventional method.

In the next step, the suspension of anti-HABP monoclonal antibody-supported latex particles was centrifuged at 15000 rpm for 15 minutes. After the supernatant solution was removed, 1 ml of 50 mM boric acid buffer (containing 2.5% BSA, pH- 7.3) was added to the pellet at the bottom of the tube for centrifugation. After that, the pellet was resuspended by ultrasonication for 1 minute under cooling with ice, and then centrifuged at 15000 rpm for 15 minutes. After the supernatant solution was removed, 1 ml of 50 mM boric acid buffer (containing 2.5% BSA, pH 7.3) was added to the pellet at the bottom of the tube for centrifugation. After that, the pellet was resuspended by ultrasonication for 1 minute under cooling with ice. Further, the suspension was incubated with stirring at room temperature for 120 minutes and the area of the latex particle surface, where no antibody has been supported, was coated with BSA.

After that, the suspension was centrifuged at 15000 rpm for 15 minutes. After the supernatant solution was removed, 1 ml of 50 mM boric acid buffer (containing 0.5% BSA, pH 7.3) was added to the pellet at the bottom of the tube for centrifugation. Then, the pellet was resuspended by ultrasonication for 1 minute under cooling with ice, and diluted 3.33 times with 50 mM boric acid buffer (containing 0.5% BSA, pH 7.3). This solution was defined as a second test solution.

### (2) Measurement of standard hyaluronic acid 1. Preparation of a standard solution of hyaluronic acid

Potassium hyaluronate (product of Wako Pure Chemical Industries, Ltd.) was diluted with 50 mM phosphate buffer (pH 7.0) to make solutions in concentration of 10, 100 and 1000 ng/ml, and used as standard solutions of hyaluronic acid.

### 2. Measurement of hyaluronic acid

The quantity of hyaluronic acid in the standard solutions of hyaluronic acid prepared in the above section 1 was measured under the condition as shown below using a fully automated measuring equipment system (JEOL Ltd.: BM-8 Model).
Sample: 10 µl
First test solution: 90 µl
Second test solution: 30 µl
Measurement method: 2 point-end method
Dominant wavelength: 571 nm

The results obtained are shown in Table 1. In this connection, the value indicated in the table is the value increased 10000 times of that obtained by subtraction of blank value (value obtained when hyaluronic acid concentration is zero) from measured value.

### Comparative Example 1

### (1) Preparation of HABP-sensitized latex particle by chemical bond, #1

To a 2 ml volume polycarbonate tube for centrifugation, 900 µl of 50 mM TAPS buffer solution (pH 8.0), 100 µl of carbonic acid latex particle solution (10 wt%; carbonic acid latex particles: 200 nm in diameter; carbonic acid content: 0.3 meq/g) and 50 µl of a 10 mg/ml aqueous solution of water soluble carbodiimide (WSC, product of Dojindo Laboratories) were added, and then left standing for 10 minutes to activate carboxyl groups on the surface of latex particles. After that, 276 µl of a 1.45 mg/ml HABP solution in ASES buffer (50 mM, pH 6.5) was added, and incubated with stirring at room temperature for 120 minutes. Additionally, 250 µl of boric acid buffer (50 mM, pH 7.3) containing 2.5% BSA was added to the reaction solution and incubated with stirring at room temperature for 60 minutes, followed by incubation at 5°C for overnight. After that, the reaction solution was centrifuged at 18000 rpm for 20 minutes. After the supernatant solution was removed, 1 ml of 50 mM boric acid buffer (containing 0.5% BSA, pH 7.3) was added to the pellet at the bottom of the tube for centrifugation. Then, the pellet was resuspended by ultrasonication for 1 minute under cooling with ice, and the same procedure was repeated twice. The solution obtained was defined as a second test solution (1).

### (2) Preparation of HABP-sensitized latex particles by chemical bond, #2

HABP-sensitized latex particles were prepared by the same procedure as described in Comparative Example 1 (1), except that the carbonic acid latex particle solution with 10wt%, 210 nm in diameter and 0.5 meq/g of carbonic acid content was used. The solution obtained was defined as second test solution (2).

### (3) Preparation of HABP-sensitized latex particle by physical adsorption

To a 2 ml volume polycarbonate tube for centrifugation, 524 µl of purified water, 100 µl of latex particle solution (N200, product of Sekisui Chemical Co., Ltd.: 10 wt%, latex particle with 220 nm in diameter), 100 µl of 500 mM boric acid buffer (pH 7.3), 276 µl of 1.45 mg/ml HABP aqueous solution were added, and incubated with stirring at room temperature for 120 minutes.
In the next step, the reaction solution was centrifuged at 15000 rpm for 15 minutes. After the supernatant solution was removed, 1 ml of 50 mM boric acid buffer (containing 2.5% BSA, pH 7.3) was added to the pellet at the bottom of the tube for centrifugation. After that, the pellet was resuspended by ultrasonication for 1 minute under cooling with ice. Then, the suspension was centrifuged at 15000 rpm for 15 minutes. After the supernatant solution was removed, 1 ml of 50 mM boric acid buffer (containing 2.5% BSA, pH 7.3) was added to the pellet at the bottom of the tube for centrifugation. Further, the pellet was resuspended by ultrasonication for 1 minute under cooling with ice. Then the suspension was incubated with stirring at room temperature for 60 minutes, and followed by incubation at 5°C overnight.
After that, the suspension was centrifuged at 15000 rpm for 15 minutes. After the supernatant solution was removed, 1 ml of 50 mM boric acid buffer (containing 0.5% BSA, pH 7.3) was added to the pellet at the bottom of the tube for centrifugation. Then, the pellet was resuspended by ultrasonication for 1 minute under cooling with ice. The solution obtained was defined as second test solution (3).

### (4) Preparation of latex particles sensitized with HABP through anti-HABP monoclonal antibody, #1

To a 2 ml volume polycarbonate tube for centrifugation, 1 ml of anti-HABP monoclonal antibody-sensitized latex particle solution prepared in Example 1 and 33. 5 µl of 896 µg/ml HABP aqueous solution were added, and incubated with stirring at room temperature for 120 minutes, and additionally incubated at 5°C for 2 days. Then, the resultant reaction solution was centrifuged at 15000 rpm for 15 minutes. After the supernatant solution was removed, 1 ml of 50 mM boric acid buffer (containing 0.5% BSA, pH 7.3) was added to the pellet at the bottom of the tube for centrifugation. Then, the pellet was resuspended by ultrasonication for 1 minute under cooling with ice. The solution obtained was defined as second test solution(4).

### (5) Comparison of calibration curve of reagents for the measurement of hyaluronic acid using each latex particle solution

The quantity of hyaluronic acid in the standard hyaluronic acid solutions was measured by the same method as (2) of Example 1, except that 100 mM HEPES buffer solution (containing 0.1% BSA and 1% NaCl, pH7.0) was used as first test solution and the second test solutions (1) to (4) were diluted by 3.33 times with 50 mM boric acid buffer (containing 0.5% BSA, pH 7.3) as second test solution.

The obtained results were shown in Table 1 together with the result of Example 1. In this connection, the value indicated in the table corresponds to the value that was obtained by subtraction of the blank value (value obtained when hyaluronic acid concentration is zero) from the measured value that was multiplied by a factor of 10000.

**Table 1**

| Concentration of hyaluronic acid (ng/ml) | Example Second TS | Second TS(1) | Second TS(2) | second TS(3) | Second TS(4) |
|---|---|---|---|---|---|
| 0 | 0 | 0 | 0 | 0 | 0 |
| 10 | 37 | 35 | 3 | -5 | 39 |
| 100 | 195 | 12 | -2 | -5 | 165 |
| 1000 | 1586 | 3 | 2 | -5 | 1579 |

| | | | | | |
|---|---|---|---|---|---|
| TS: test solution | | | | | |

As is clear from the results shown in Table 1, when the HABP-sensitized latex particle prepared either by chemical bond (second test solution(1) and (2) in Table 1) and by physical adsorption (second test solution (3) in Table 1) were used, agglutination of latex particles corresponding to the concentration of hyaluronic acid was not observed, and thus accurate measurement could not be performed. In addition, no significant difference in the measurement was observed, between the case when a test solution containing the particle, wherein HABP is preliminarily bound to anti-HABP monoclonal antibody-supported latex particle, was used as second test solution (second test solution (4) in Table 1) and the case when a solution containing HABP was used as first test solution and a solution containing anti-HABP monoclonal antibody-supported latex particle was used as second test solution (second test solution of Example in Table 1), and thus, it was confirmed that, by the use of these methods, a highly accurate measurement of hyaluronic acid can be performed.

### Experimental Example 1

### Comparison of temporal stability of test solutions

The first test solution and the second test solution were stored at 30°C for 1 month, and then the quantity of hyaluronic acid was measured by the same procedure as described in Example 1 (2) using these stored test solutions. In addition, both the first test solution and the second test solution(4) used in Comparative Example 1 were stored at 30°C for 1 month, and then the quantity of hyaluronic acid was measured by the same procedure as described in Comparative Example 1 (5) using these stored test solutions.

The results obtained were shown in Table 2. In this connection, the absorbance value indicated in the table corresponds to the value that was obtained by subtraction of the blank value (value obtained when hyaluronic acid concentration is zero) from the measured value that was multiplied by a factor of 10000. In addition, absorbance retention was expressed as the percentage of the value obtained by dividing the absorbance measured after incubation for 1 month by the absorbance measured before incubation.

**Table 2**

| Concentration of hyaluronic acid, (ng/ml) | Example | | Second test solution (4) | |
|---|---|---|---|---|
| | Absorbance | Absorbance retention after 1 month (%) | Absorbance | Absorbance retention after 1 month (%) |
| 0 | 0 | 0 | 0 | 0 |
| 100 | 116 | 80 | 0 | 0 |
| 1000 | 1293 | 95 | 164 | 10 |
| 5000 | 3529 | 90 | 449 | 10 |
| 10000 | 2850 | 85 | 529 | 12 |
| Average | - | 87 | - | 8 |

As is clear from the results shown in Table 2, when the second test solution(4), namely, a test solution containing the particle, wherein HABP is preliminarily bound to anti-HABP monoclonal antibody-supported latex particle, was used as second test solution, the absorbance after passing for 1 month, as compared to that of before passage, has decreased obviously. On the other hand, when test solutions in Example 1 were used, namely, a solution containing HABP was used as first test solution and a solution containing anti-HABP monoclonal antibody-supported latex particle was used as second test solution, the absorbance retention was 80% or more, and the stability of the reagent was found to be significantly high compared to that observed for the second test solution(4). Thus, it was confirmed that, by the use of a reagent kit of the present invention, a highly accurate measurement of hyaluronic acid can be performed even if the reagents are stored for long time.

## Claims

1. A method for measuring hyaluronic acid comprising:
forming a hyaluronic acid/hyaluronic acid binding protein complex by contacting hyaluronic acid in a sample with hyaluronic acid binding protein,
reacting said complex with an anti-hyaluronic acid binding protein antibody-supported latex particle whose diameter is 0.05 to 0.3 µm,
measuring optical change by agglutination product generated by said reaction and
calculating the quantity of hyaluronic acid from the measured value.

2. The method according to claim 1, wherein the hyaluronic acid binding protein is one selected from proteoglycan, link protein and hyaluronectin.

3. The method according to claim 1, wherein the optical change is the change of turbidity or the change of absorbance, or the change of scattered light intensity.

4. A reagent kit for the measurement of hyaluronic acid according to the method of any one of claims 1 to 3 comprising a reagent comprising a hyaluronic acid binding protein and a reagent comprising an anti-hyaluronic acid binding protein antibody-supported latex particle whose diameter is 0.05 to 0.3 µm.

## Patentansprüche

1. Verfahren zum Messen von Hyaluronsäure, umfassend:
Bilden eines Komplexes aus Hyaluronsäure/Hyaluronsäure-bindendem Protein durch In-Kontakt-Bringen von Hyaluronsäure in einer Probe mit Hyaluronsäure-bindendem Protein;
Umsetzen des Komplexes mit einem Anti-Hyaluronsäure-bindendes-Protein-Antikörper auf einem Latexteilchen, dessen Durchmesser 0,05 bis 0,3 µm beträgt, als Träger;
Messen der optischen Veränderung durch Agglutinierungsprodukt, das durch die Reaktion entstanden ist; und
Berechnen der Menge an Hyaluronsäure aus dem gemessenen Wert.

2. Verfahren gemäß Anspruch 1, wobei das Hyaluronsäure-bindende Protein aus Proteoglycan, Link-Protein und Hyaluronektin ausgewählt ist.

3. Verfahren gemäß Anspruch 1, wobei es sich bei der optischen Veränderung um eine Änderung der Trübheit oder eine Änderung der Extinktion oder eine Änderung der Streulichtintensität handelt.

4. Reagens-Kit zur Messung von Hyaluronsäure nach dem Verfahren gemäß einem der Ansprüche 1 bis 3, umfassend ein Reagens, das ein Hyaluronsäure-bindendes Protein umfasst, und ein Reagens, das einen Anti-Hyaluronsäure-bindendes-Protein-Antikörper auf einem Latexteilchen, dessen Durchmesser 0,05 bis 0,3 µm beträgt, als Träger umfasst.

## Revendications

1. Procédé pour mesurer l'acide hyaluronique comprenant :
la formation d'un complexe acide hyaluronique / protéine de liaison à l'acide hyaluronique en mettant en contact l'acide hyaluronique d'un échantillon avec la protéine de liaison à l'acide hyaluronique,
la réaction dudit complexe avec une particule de latex sur laquelle est fixé un anticorps anti-protéine de liaison à l'acide hyaluronique dont le diamètre est de 0,05 à 0,3 µm,
la mesure de la modification optique due au produit d'agglutination résultant de ladite réaction et
le calcul de la quantité d'acide hyaluronique à partir de la valeur mesurée.

2. Procédé selon la revendication 1, dans lequel la protéine de liaison à l'acide hyaluronique est sélectionnée parmi le protéoglycane, la protéine « link » et l'hyaluronectine.

3. Procédé selon la revendication 1, dans lequel la modification optique est la modification de turbidité ou la modification d'absorbance, ou la modification de l'intensité de la lumière diffusée.

4. Kit de réactifs destiné à mesurer l'acide hyaluronique selon le procédé selon l'une quelconque des revendications 1 à 3, contenant un réactif contenant une protéine de liaison à l'acide hyaluronique et un réactif contenant une particule de latex sur laquelle est fixé l'anticorps anti-protéine de liaison à l'acide hyaluronique dont le diamètre est de 0,05 à 0,3 µm.
